# EUROPEAN PATENT APPLICATION

(11) **EP 4 394 037 A1**
(43) Date of publication of application: **03.07.2024**
(21) Application number: 22861329.5
(22) Date of filing: 22.08.2022
(51) Int. Cl.: C12N 15/09, A01K 67/027, C07K 14/195, C07K 19/00, C12N 1/15, C12N 1/19, C12N 1/21, C12N 5/10, C12N 9/22, C12N 15/55, C12N 15/62, C12N 15/63, C12P 21/02

(54) **SITE-SPECIFIC NUCLEASE**

(30) Priority: 23.08.2021 JP 2021135502
(71) Applicant: GRA&GREEN Inc., Nagoya-shi, Aichi 464-0807 (JP)
(72) Inventor: KOBAYASHI, Takehito, Nagoya-shi, Aichi 464-0814 (JP)
(74) Representative: Hoffmann Eitle
(86) International application number: PCT/JP2022/031615
(87) International publication number: WO 2023/027041

(57) **Abstract**

Provided is a genome editing technique that achieves higher genome editing efficiency. Any of Proteins 1 to 3 of the present invention.

## Description

### Technical Field

The present invention relates to, for example, a site-specific nuclease.

### Background Art

Development and improvement of a CRISPR/Cas system have been rapidly progressing. According to this system, the combination of a guide RNA and a Cas protein can cause DNA double-strand breaks at any site of the genome of a cell. After double-strand breaks occur in a gene, DNA mutations, such as random deletion and addition, may occur during repair by non-homologous end joining at the cleaved ends, causing knock-out of the gene by a frameshift etc.

As the Cas protein, Cas9 protein from *S. pyogenes* (SpCas9) has been widely used. Novel Cas proteins, such as Cpf1 protein and MAD7 protein, have recently been reported. Since the genome editing efficiency of these Cas proteins may be unsatisfactory compared with SpCas9, various techniques to attempt to improve genome editing efficiency have been reported. For example, Non-patent Literature (NPL) 1 discloses that an attempt has been made to improve genome editing efficiency by linking a nuclear localization signal to Cas12a protein.

### Citation List

### Non-patent Literature

NPL 1: Liu P, Luk K, Shin M, et al. Enhanced Cas12a editing in mammalian cells and zebrafish. Nucleic Acids Res. 2019; 47(8):4169-4180. doi:10.1093/nar/gkz184

### Summary of Invention

### Technical Problem

A problem to be solved by the present invention is to provide a genome editing technique that achieves higher genome editing efficiency.

### Solution to Problem

As a result of extensive research in view of the above problem, the present inventor found that any one of Proteins 1 to 3 of the present invention, which are described later, can solve the above problem. After further research based on such findings, the inventor has completed the present invention. More specifically, the present invention encompasses the following embodiments.

Item 1. A protein comprising
an amino acid sequence b1 of MAD7 protein,
wherein the amino acid sequence b1 is a mutant sequence of the amino acid sequence represented by SEQ ID NO: 6 and comprises a K169R mutation in the amino acid sequence represented by SEQ ID NO: 6.

Item 2. The protein according to Item 1, wherein the amino acid sequence b1 further comprises at least one mutation selected from the group consisting of S469R and H1025K in the amino acid sequence represented by SEQ ID NO: 6.

Item 3. The protein according to Item 1 or 2, comprising
(b) a MAD7 domain comprising the amino acid sequence b1 of MAD7 protein and a concatenated sequence b2 of multiple nuclear localization signal sequences.

Item 4. The protein according to Item 3, wherein in the MAD7 domain, the concatenated sequence b2 is located on the C-terminal side of the amino acid sequence b1.

Item 5. The protein according to Item 3 or 4, comprising:
(a) a 5' to 3' exonuclease domain comprising an amino acid sequence a1 of 5' to 3' exonuclease and a nuclear localization signal sequence a2; and
(b) a MAD7 domain comprising the amino acid sequence b1 of MAD7 protein and a concatenated sequence b2 of multiple nuclear localization signal sequences.

Item 6. The protein according to Item 5, wherein the 5' to 3' exonuclease domain is located on the N-terminal side of the MAD7 domain.

Item 7. The protein according to Item 5 or 6, wherein in the 5' to 3' exonuclease domain, the nuclear localization signal sequence a2 is located on the N-terminal side of the amino acid sequence a1.

Item 8. The protein according to any one of Items 5 to 7, wherein the nuclear localization signal sequence a2, the amino acid sequence a1, the amino acid sequence b1, and the concatenated sequence b2 are arranged in this order from the N-terminal side.

Item 9. The protein according to any one of Items 1 to 8, wherein the amino acid sequence b1 is a mutant sequence of the amino acid sequence represented by SEQ ID NO: 6 and comprises at least one mutation selected from the group consisting of A24E, I180K, A290R, S469K, S469R, K535R, N583K, N583R, K590R, I646K, I646R, T714K, T714R, N827D, Y832K, Y832R, K970R, H1025K, H1025R, S1175A, and C1219N in the amino acid sequence represented by SEQ ID NO: 6.

Item 10. The protein according to Item 9, wherein the mutation is at least one mutation selected from the group consisting of N583K, N583R, Y832K, Y832R, H1025K, H1025R, and C1219N.

Item 11. A protein comprising:
(a) a 5' to 3' exonuclease domain comprising an amino acid sequence a1 of 5' to 3' exonuclease and a nuclear localization signal sequence a2; and
(b) a MAD7 domain comprising an amino acid sequence b1 of MAD7 protein and a concatenated sequence b2 of multiple nuclear localization signal sequences.

Item 12. A protein comprising
an amino acid sequence b1,
wherein the amino acid sequence b1 is a mutant sequence of the amino acid sequence represented by SEQ ID NO: 6 and comprises at least one mutation selected from the group consisting of A24E, K169R, I180K, A290R, S469K, S469R, K535R, N583K, N583R, K590R, I646K, I646R, T714K, T714R, N827D, Y832K, Y832R, K970R, H1025K, H1025R, S1175A, and C1219N in the amino acid sequence represented by SEQ ID NO: 6.

Item 13. A protein comprising:
(ai) a 5' to 3' exonuclease domain comprising an amino acid sequence a1 of 5' to 3' exonuclease and a nuclear localization signal sequence a2; and
(bi) a MAD7 domain comprising an amino acid sequence b1 of MAD7 protein.

Item 14. A polynucleotide comprising a coding sequence for the protein of any one of Items 1 to 13.

Item 15. A polynucleotide comprising:
(aii) an expression cassette of a protein comprising a 5' to 3' exonuclease domain comprising an amino acid sequence a1 of 5' to 3' exonuclease and a nuclear localization signal sequence a2; and
(bii) an expression cassette of a protein comprising a MAD7 domain comprising an amino acid sequence b1 of MAD7 protein and a concatenated sequence b2 of multiple nuclear localization signal sequences.

Item 16. A cell comprising the polynucleotide of Item 14 or 15.

Item 17. A composition for genome editing, comprising at least one member selected from the group consisting of the protein of any one of Items 1 to 13 and the polynucleotide of Item 14 or 15.

Item 18. A genome editing method, comprising introducing at least one member selected from the group consisting of the protein of any one of Items 1 to 13 and the polynucleotide of Item 14 or 15 into a cell or a non-human organism.

Item 19. A method for producing a genome-edited cell or genome-edited non-human organism, comprising introducing at least one member selected from the group consisting of the protein of any one of Items 1 to 13 and the polynucleotide of Item 14 or 15 into a cell or a non-human organism.

### Advantageous Effects of Invention

The present invention can provide a genome editing technique that achieves higher genome editing efficiency.

### Brief Description of Drawings

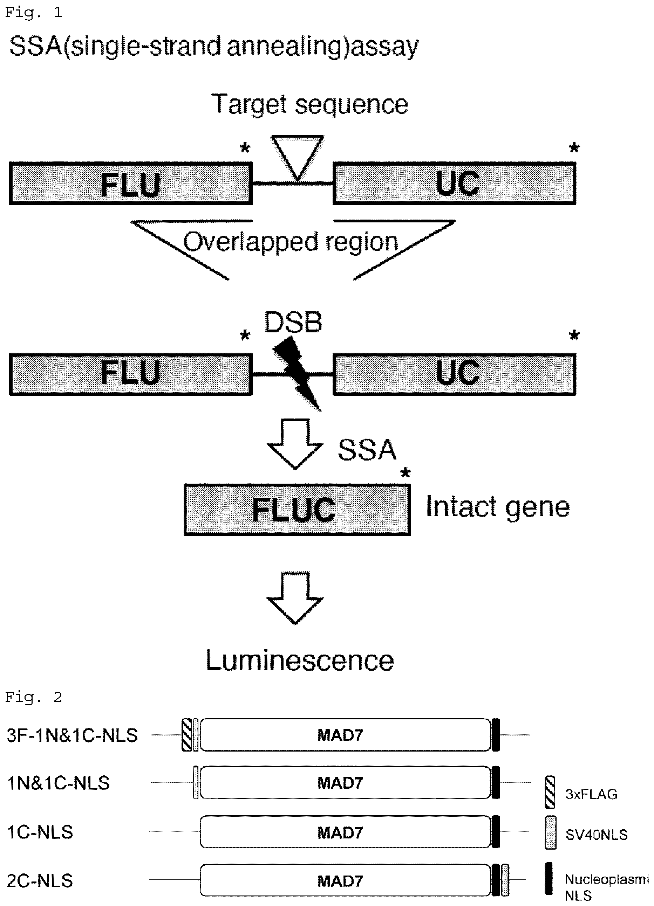

Fig. 1 shows an overview of SSA assay (an assay to measure genome editing efficiency). "∇" indicates the location of the target sequence for genome editing. "*" indicates a stop codon.
Fig. 2 shows the configurations of effector expression vectors used in Test Example 1-1.

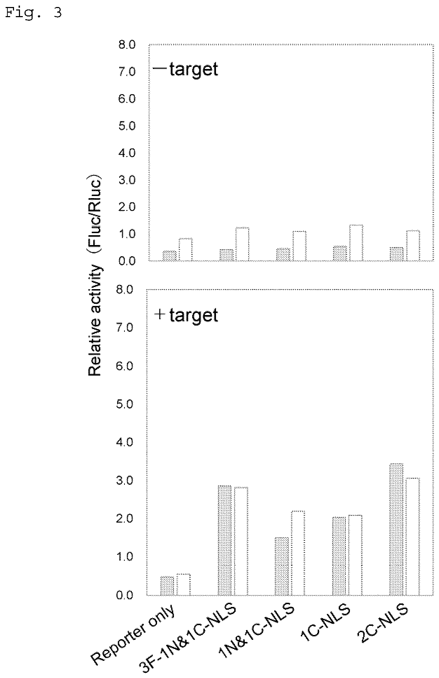

Fig. 3 is graphs showing Fluc/Rluc values obtained with the use of each effector expression vector in Test Example 1-1. On the horizontal axis, "Reporter only" indicates the case in which no effector expression vector was used, and the others indicate cases in which an effector expression vector (the names and configurations correspond to those shown in Fig. 2) was used. The upper graph (-target) shows the results of cases in which pALTER-pSSA (a vector without the target sequence of guide RNA) was used as the reporter vector, and the lower graph (+target) shows the results of cases in which pALTER-pSSA-NbPDS (a vector with the target sequence of guide RNA) was used as the reporter vector. Dark-gray and light-gray columns represent the results of independently conducted tests.

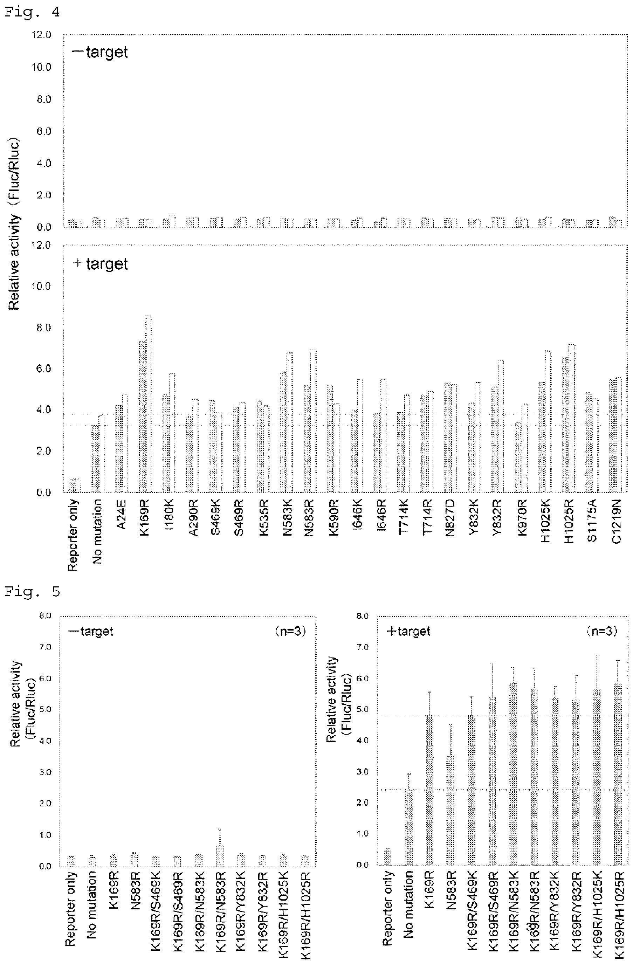

Fig. 4 is graphs showing Fluc/Rluc values obtained with the use of each effector expression vector in Test Example 1-2. On the horizontal axis, "Reporter only" indicates the case in which no effector expression vector was used, "No mutation" indicates the case in which no mutation was introduced into MAD7 (when pALTER-MAD7-2xNLS was used), and the others indicate mutations introduced into MAD7. The upper and lower graphs and dark-gray and light-gray columns are as described in Fig. 3.
Fig. 5 is graphs showing Fluc/Rluc values obtained with the use of each effector expression vector in Test Example 1-3. On the horizontal axis, "Reporter only" indicates the case in which no effector expression vector was used, "No mutation" indicates the case in which no mutation was introduced into MAD7 (when pALTER-MAD7-2xNLS was used), and the others indicate mutations introduced into MAD7. Each column represents the average values of three independently conducted tests. The left and right graphs are as described in Fig. 3.

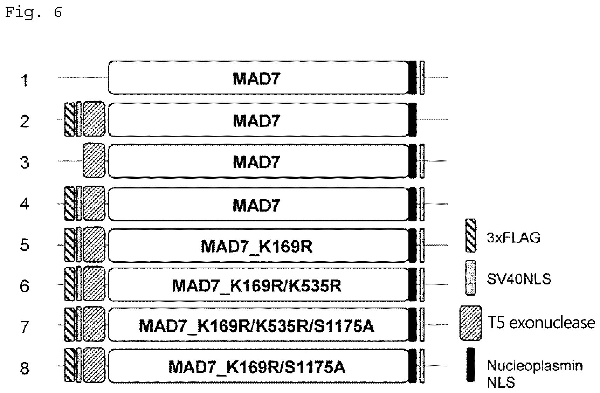

Fig. 6 shows the configurations of effector expression vectors used in Test Example 1-4.

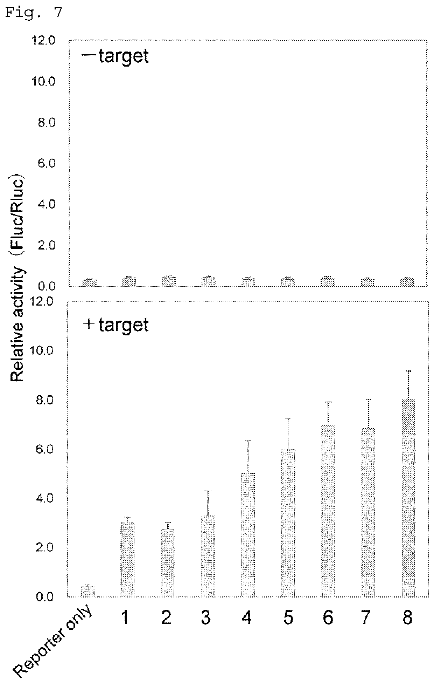

Fig. 7 is graphs showing Fluc/Rluc values obtained with the use of each effector expression vector in Test Example 1-4. On the horizontal axis, "Reporter only" indicates the case in which no effector expression vector was used, and the others indicate cases in which an effector expression vector (the names and configurations correspond to those shown in Fig. 6) was used. Each column represents the average values of three independently conducted tests. The upper and lower graphs are as described in Fig. 3.

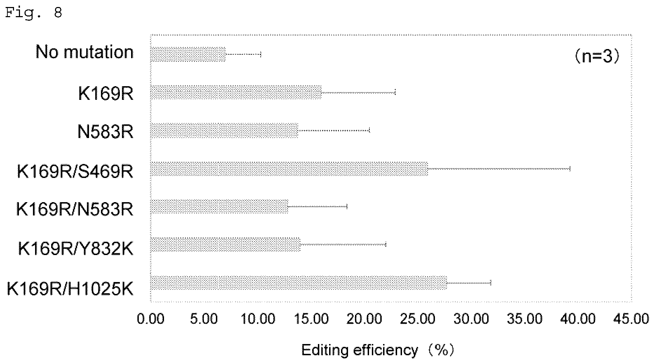

Fig. 8 is a graph showing genome editing efficiency (%) as measured and calculated in Test Example 2. On the vertical axis, "No mutation" indicates the case in which no mutation was introduced into MAD7, and the others indicate mutations introduced into MAD7. Each column represents the average values of three independently conducted tests.

### Description of Embodiments

### 1. Definition

In the present specification, the terms "comprising," "containing," "including," and "having" include the concepts of containing, including, consisting essentially of, and consisting of.

In the present specification, the "identity" of amino acid sequences refers to the degree of identicalness of two or more amino acid sequences that can be compared with each other. Thus, the higher the identicalness of two amino acid sequences, the higher the identity or similarity of these sequences. The level of amino acid sequence identity is determined by, for example, using FASTA, which is a tool for sequence analysis, with default parameters. The level of amino acid sequence identity can otherwise be determined by using the algorithm BLAST by Karlin and Altschul (Karlin S, Altschul SF, "Methods for assessing the statistical significance of molecular sequence features by using general scoring schemes," Proc Natl Acad Sci USA, 87: 2264-2268 (1990); Karlin S, Altschul SF, "Applications and statistics for multiple high-scoring segments in molecular sequences," Proc Natl Acad Sci USA, 90: 5873-7 (1993)). Programs called "blastp" and "tblastn," which are based on such an algorithm of BLAST, have been developed. Specific procedures for these analysis methods are known, and reference may be made to the National Center of Biotechnology Information (NCBI) website (http://www.ncbi.nlm.nih.gov/). The "identity" of base sequences is also defined accordingly.

In the present specification, "conservative substitution" means a substitution of an amino acid residue with an amino acid residue having a similar side chain. For example, a substitution between amino acid residues having a basic side chain, such as lysine, arginine, and histidine, is considered to be a conservative substitution. Other examples that are considered to be a conservative substitution include a substitution between amino acid residues having an acidic side chain, such as aspartic acid and glutamic acid; a substitution between amino acid residues having an uncharged polar side chain, such as asparagine, glutamine, serine, threonine, tyrosine, and cysteine; a substitution between amino acid residues having a nonpolar side chain, such as glycine, alanine, valine, leucine, isoleucine, proline, phenylalanine, methionine, and tryptophan; a substitution between amino acid residues having a branched chain, such as valine, isoleucine, and leucine; and a substitution between amino acid residues having an aromatic side chain, such as tyrosine, phenylalanine, and tryptophan.

In the present specification, the "coding sequence" is not particularly limited as long as it is a base sequence encoding the amino acid sequence of a protein.

In the present specification, an amino acid mutation in a specific amino acid sequence may be represented by a single-letter code for an amino acid before mutation + the position number of the amino acid counted from the amino acid of the N-terminus + a single-letter code for the amino acid after mutation (mutation name) in the specific amino acid sequence. For example, the mutation name "K169R" in the amino acid sequence represented by SEQ ID NO: 6 indicates a mutation of lysine (K), which is the amino acid at position 169 counted from the amino acid of the N-terminus in the amino acid sequence represented by SEQ ID NO: 6, to arginine (R). When multiple mutations are provided, each mutation name of the multiple mutations may be listed together with a slash between them. For example, the mutation name "K169R/K535R" in the amino acid sequence represented by SEQ ID NO: 6 indicates that K169R and K535R mutations are both provided.

In the present specification, "domain" refers to a region that constitutes a portion of a protein and comprises contiguous amino acid sequences.

### 2. Protein

In one embodiment, the present invention relates to a protein comprising (a) a 5' to 3' exonuclease domain comprising an amino acid sequence a1 of 5' to 3' exonuclease and a nuclear localization signal sequence a2, and (b) a MAD7 domain comprising an amino acid sequence b1 of MAD7 protein and a concatenated sequence b2 of multiple nuclear localization signal sequences (which may be referred to herein as "Protein 1 of the present invention"). This protein is described below.

Protein 1 of the present invention comprises a 5' to 3' exonuclease domain and a MAD7 domain.

The 5' to 3' exonuclease domain is a domain comprising an amino acid sequence a1 of 5' to 3' exonuclease and a nuclear localization signal sequence a2.

The amino acid sequence a1 is not particularly limited as long as it is the amino acid sequence of a 5' to 3' exonuclease. The 5' to 3' exonuclease is not particularly limited as long as it is a protein having 5' to 3' exonuclease activity, i.e., the activity to cleave nucleic acids (DNA) sequentially from the 5' end. Examples include T5 exonuclease from phage (specifically, for example, the amino acid sequence SEQ ID NO: 64), a 5' to 3' exonuclease from *Candidatus Pelagibacter sp.* (specifically, for example, Genbank ID MAJ58196.1, the amino acid sequence SEQ ID NO: 65), a 5' to 3' exonuclease from Betaproteobacteria bacterium (specifically, for example, Genbank ID NDC03965.1, the amino acid sequence SEQ ID NO: 66), a 5' to 3' exonuclease from Pelagibacterales bacterium (specifically, for example, Genbank ID MBL6840569.1, the amino acid sequence SEQ ID NO: 67), a 5' to 3' exonuclease from *Marinovum sp.* (specifically, for example, Genbank ID MAJ03254.1, the amino acid sequence SEQ ID NO: 68), a 5' to 3' exonuclease from *Agrobacterium fabrum* (specifically, for example, Genbank ID WP_144623114.1, the amino acid sequence SEQ ID NO: 69), a 5' to 3' exonuclease from *Bacteriophage Eos* (specifically, for example, Genbank ID QGH45232.1, the amino acid sequence SEQ ID NO: 70), a 5' to 3' exonuclease from *Providencia phage vB_PreS_PR1* (specifically, for example, Genbank ID YP_009599164.1, the amino acid sequence SEQ ID NO: 71), a 5' to 3' exonuclease from *Pantoea phage vB_PagS_AAS21* (specifically, for example, Genbank ID QCW23761.1, the amino acid sequence SEQ ID NO: 72), a 5' to 3' exonuclease from *Klebsiella phage vB_KaS-Veronica* (specifically, for example, Genbank ID CAD5240202.1, the amino acid sequence SEQ ID NO: 73), a 5' to 3' exonuclease from *Proteus phage PM135* (specifically, for example, Genbank ID YP_009620590.1, the amino acid sequence SEQ ID NO: 74), a 5' to 3' exonuclease from *Pectobacterium phage My1* (specifically, for example, Genbank ID YP_006906376.1, the amino acid sequence SEQ ID NO: 75), a 5' to 3' exonuclease from *Vibrio phage pVp-1* (specifically, for example, Genbank ID YP_007007826.1, the amino acid sequence SEQ ID NO: 76), a 5' to 3' exonuclease from *Aeromonas phage AhSzw-1* (specifically, for example, Genbank ID YP_009800308.1, the amino acid sequence SEQ ID NO: 77), a 5' to 3' exonuclease from *Phyllobacterium myrsinacearum* (specifically, for example, Genbank ID WP_182552320.1, the amino acid sequence SEQ ID NO: 78), a 5' to 3' exonuclease from *Rhizobium azibense* (specifically, for example, Genbank ID TDW20525.1, the amino acid sequence SEQ ID NO: 79), and the like. Of these, particularly preferred examples include T5 exonuclease.

The 5' to 3' exonuclease may comprise one or more amino acid sequence mutations (e.g., substitution, deletion, insertion, and addition) as long as the activity is not significantly impaired. From this perspective, the 5' to 3' exonuclease may be a protein comprising an amino acid sequence having, for example, 70% or more, preferably 80% or more, more preferably 90% or more, even more preferably 95% or more, still more preferably 97% or more, and particularly preferably 99% or more identity to the amino acid sequence of a wild-type 5' to 3' exonuclease, and having the activity thereof. The above "activity" can be evaluated in vitro or in vivo according to or in accordance with known methods.

The amino acid sequence a1 is preferably, for example, an amino acid sequence having 70% or more identity to the amino acid sequences represented by SEQ ID NOs: 64 to 79.

The nuclear localization signal sequence a2 is not particularly limited as long as it is an amino acid sequence of a nuclear localization signal. The nuclear localization signal is an amino acid sequence that can target a protein of interest to the nucleus; in other words, the nuclear localization signal labels a protein of interest for import into the nucleus. Targeting to the nucleus is made possible, for example, by nuclear localization signals binding to their receptors, which are known as importins (karyopherins). Protein transport into the nucleus is typically initiated by the formation of a ternary complex of importin α, importin β1, and cargo (e.g., a polypeptide). Importin β1 leads the complex into the nucleus by docking it with the nuclear membrane pore complex and releases cargo into the nucleus through binding of Ran-GTP to importin β1. In the importin α/β pathway, importin α acts as an adapter that links cargo to importin β1 and recognizes nuclear localization signals in cargo. Typically, a nuclear localization signal can comprise or consist of a short sequence (e.g., 2 to 10, 3 to 8, or 4 to 6 residues) of one or more positively charged amino acids (e.g., lysine and arginine). Various amino acid sequences are known as nuclear localization signals. Examples of nuclear localization signals include a classical NLS (cNLS), which comprises one (monopartite) or two (bipartite) continuous sequences of basic amino acids. A monopartite nuclear localization signal sequence is typically characterized by a sequence of a helix-breaking residue (e.g., proline, glycine) and a basic residue (e.g., 2 to 10, 3 to 8, or 4 to 6 residues) (or a consensus sequence K(K/R)X(K/R) wherein X is any amino acid) arranged in this order. A bipartite nuclear localization signal sequence is typically characterized by a basic-residue sequence (e.g., 2 to 10, 3 to 8, 4 to 6 residues), a linker sequence, and a basic-residue sequence (e.g., 2 to 10, 3 to 8, 4 to 6 residues) (or a consensus sequence R/K(X)₁₀₋₁₂KRXK wherein X is any amino acid) arranged in this order. Specific examples of monopartite nuclear localization signal sequences include an SV40 nuclear localization signal sequence (amino acid sequence: SEQ ID NO: 10). Specific examples of bipartite nuclear localization signal sequences include a nucleoplasmin nuclear localization signal (amino acid sequence: SEQ ID NO: 12).

The nuclear localization signal sequence may comprise one or more amino acid sequence mutations (e.g., substitution, deletion, insertion, and addition) as long as the activity is not significantly impaired. From this perspective, the nuclear localization signal sequence may be a sequence comprising an amino acid sequence having, for example, 70% or more, preferably 80% or more, more preferably 90% or more, even more preferably 95% or more, still more preferably 97% or more, and particularly preferably 99% or more identity to the amino acid sequence of a wild-type nuclear localization signal sequence, and having the activity thereof. The above "activity" can be evaluated in vitro or in vivo according to or in accordance with known methods.

In one embodiment of the present invention, the nuclear localization signal sequence a2 is preferably a monopartite nuclear localization signal sequence.

The nuclear localization signal sequence a2 may consist of a single nuclear localization signal sequence, or may be a sequence of multiple (e.g., 2 to 6, 2 to 5, 2 to 4, 2 to 3) identical or different nuclear localization signal sequences linked together. In the latter, the nuclear localization signal sequences may be directly linked to each other not via an additional amino acid sequence or may be linked via an additional amino acid sequence (e.g., an amino acid sequence of about 1 to 50, 1 to 30, 1 to 20, 1 to 10, or 1 to 5 residues (e.g., a linker sequence)). In one embodiment of the present invention, the nuclear localization signal sequence a2 preferably consists of a single nuclear localization signal sequence.

In the 5' to 3' exonuclease domain, the linkage mode between the amino acid sequence a1 and the nuclear localization signal sequence a2 is not particularly limited and may be directly linked to each other not via an additional amino acid sequence or may be linked via an additional amino acid sequence (e.g., an amino acid sequence of about 1 to 50, 1 to 30, 1 to 20, 1 to 10, or 1 to 5 residues (e.g., a linker sequence)).

The positional relationship between the amino acid sequence a1 and the nuclear localization signal sequence a2 in the 5' to 3' exonuclease domain is not particularly limited, and includes both an embodiment in which the nuclear localization signal sequence a2 is located on the N-terminal side of the amino acid sequence a1 and an embodiment in which the nuclear localization signal sequence a2 is located on the C-terminal side of the amino acid sequence a1. In one embodiment of the present invention, the nuclear localization signal sequence a2 is preferably located on the N-terminal side of the amino acid sequence a1. In one embodiment of the present invention, when the 5' to 3' exonuclease domain is located on the N-terminal side of the MAD7 domain, the nuclear localization signal sequence a2 is preferably located on the N-terminal side of the amino acid sequence a1. On the other hand, when the 5' to 3' exonuclease domain is located on the C-terminal side of the MAD7 domain, the nuclear localization signal sequence a2 is preferably located on the C-terminal side of the amino acid sequence a1.

The MAD7 domain is a domain comprising an amino acid sequence b1 of MAD7 protein and a concatenated sequence b2 of multiple nuclear localization signal sequences.

The amino acid sequence b1 is not particularly limited as long as it is an amino acid sequence of MAD7 protein. MAD7 protein is Cas12a protein from *Eubacterium rectale.* MAD7 protein can be used in the CRISPR/Cas system and, for example, can bind to and cleave a target site of genomic DNA while forming a complex with guide RNA. The information on the amino acid sequence of MAD7 protein and its coding sequence can be easily obtained from various databases, such as NCBI. Typically, the amino acid sequence of MAD7 protein is, for example, the amino acid sequence represented by SEQ ID NO: 6.

The MAD7 protein may comprise one or more amino acid sequence mutations (e.g., substitution, deletion, insertion, and addition) as long as the activity is not significantly impaired. From this perspective, the MAD7 protein may be a protein comprising an amino acid sequence having, for example, 70% or more, preferably 80% or more, more preferably 90% or more, even more preferably 95% or more, still more preferably 97% or more, and particularly preferably 99% or more identity to the amino acid sequence represented by SEQ ID NO: 6, and having the activity thereof. The above "activity" can be evaluated in vitro or in vivo according to or in accordance with known methods.

The amino acid sequence b1 is preferably an amino acid sequence having 70% or more identity to the amino acid sequence represented by SEQ ID NO: 6.

In a preferred embodiment of the present invention, the amino acid sequence b1 is a mutant sequence of the amino acid sequence represented by SEQ ID NO: 6 and can comprise at least one mutation selected from the group consisting of A24E, K169R, I180K, A290R, S469K, S469R, K535R, N583K, N583R, K590R, I646K, I646R, T714K, T714R, N827D, Y832K, Y832R, K970R, H1025K, H1025R, S1175A, and C1219N in the amino acid sequence represented by SEQ ID NO: 6. Genome editing efficiency can thereby be further improved. In a further preferred embodiment, the mutation above can be at least one mutation selected from the group consisting of K169R, N583K, N583R, Y832K, Y832R, H1025K, H1025R, and C1219N. In a further preferred embodiment, the mutation above can be K169R.

The combination of two mutations is preferably K169R/S469R, K169R/N583K, K169R/N583R, K169R/Y832K, K169R/Y832R, K169R/H1025R, or K169R/H1025K. The combination of three mutations is preferably K169R/N583K/H1025R, K169R/N583K/H1025K, K169R/N583R/H1025R, K169R/N583R/H1025K, K169R/N583K/Y832K, K169R/N583K/Y832R, K169R/N583K/C1219N, K169R/N583K/I646R, K169R/N583K/S469R, K169R/N583K/N827D, K169R/N583R/Y832K, K169R/N583R/Y832R, K169R/N583R/C1219N, K169R/N583R/I646R, K169R/N583R/S469R, K169R/N583R/N827D, K169R/H1025R/C1219N, K169R/H1025R/Y832K, K169R/H1025R/Y832R, K169R/H1025R/I646R, K169R/H1025R/S469R, K169R/H1025R/N827D, or K169R/H1025K/S469R. Other examples of MAD7 protein mutants are found in WO2021/257716, WO2020/086475, and Japanese Patent No. 7113415. The mutations can be combined with the mutations disclosed in these documents. In a particularly preferred embodiment of the present invention, from the perspective of genome editing efficiency (in particular, genome editing efficiency in plant cells), the amino acid sequence b1 is a mutant sequence of the amino acid sequence represented by SEQ ID NO: 6 and preferably comprises a K169R mutation in the amino acid sequence represented by SEQ ID NO: 6, and more preferably further comprises, in addition to the K169R mutation, at least one mutation selected from the group consisting of S469R and H1025K. In one embodiment of the present invention, an amino acid sequence of a Cas protein other than MAD7 protein (an amino acid sequence b1') may be used instead of the amino acid sequence b1.

Cas proteins are not particularly limited as long as they are those that are used in the CRISPR/Cas system. For example, various types of Cas proteins that can bind to and cleave a target site of genomic DNA while forming a complex with guide RNA can be used. Known Cas proteins include those from various organisms. Examples include a Cas9 protein (type II-A) from *S. pyogenes,* a Cas9 protein from *S. thermophilus,* a Cas9 protein from *S. agalactiae,* a Cas9 protein from *S. aureus,* a Cas9 protein from *N. meningitidis,* a Cas9 protein from *T. denticola,* a Cas protein (type I-A) from *S. solfataricus,* a Cas protein (type I-B) from *H. walsbyi,* a Cas protein (type I-D) from *Microcystis aeruginosa,* a Cas protein (type I-E) from *E*. *coli,* a Cas protein (type I-F) from *E*. *coli,* a Cas protein (type I-F) from P. *aeruginosa,* a Cpf1 protein (type V-A) from *F*. *novicida,* and the like. Of these, preferred examples include Cas9 proteins, and more preferred examples include Cas9 proteins endogenously found in bacteria belonging to the genus *Streptococcus.* The information on the amino acid sequences of various Cas proteins and their coding sequences can be easily obtained from various databases, such as NCBI.

The Cas protein may be a wild-type double-strand break-generating Cas protein or a Nickase Cas protein. The double-strand break-generating Cas protein usually comprises a domain involved in cleavage of target strand (HNH domain) and a domain involved in cleavage of non-target strand (RuvC domain). The Nickase Cas protein may be, for example, a protein comprising a mutation that reduces the cleavage activity of one of the two domains of a double-strand break-generating Cas protein (e.g., reduces the cleavage activity to 1/2, 1/5, 1/10, 1/100, or 1/1000 or less). For example, if the double-strand break-generating Cas protein is a Cas9 protein from *S. pyogenes,* examples of such a mutation include a mutation of the 10th amino acid (aspartic acid) from the N-terminus to alanine (D10A: a mutation in the RuvCI domain), a mutation of the 840th amino acid (histidine) from the N-terminus to alanine (H840A: a mutation in the HNH domain), a mutation of the 863rd amino acid (asparagine) from the N-terminus to alanine (N863A: a mutation in the HNH domain), a mutation of the 762nd amino acid (glutamic acid) from the N-terminus to alanine (E762A: a mutation in the RuvCII domain), a mutation of the 986th amino acid (aspartic acid) from the N-terminus to alanine (D986A: a mutation in the RuvCIII domain), and the like.

The Cas protein may comprise one or more amino acid sequence mutations (e.g., substitution, deletion, insertion, and addition) as long as the activity is not impaired. From this perspective, the Cas protein may be a protein comprising an amino acid sequence having, for example, 85% or more, preferably 90% or more, more preferably 95% or more, and even more preferably 98% or more identity to the amino acid sequence of a wild-type double-strand break-generating Cas protein or a Nickase Cas protein based on the wild-type double-strand break-generating Cas protein, and having the activity thereof (the activity to bind to and cleave a target site of genomic DNA while forming a complex with guide RNA). Alternatively, from the same perspective, the Cas protein may be a protein comprising an amino acid sequence in which one or more (e.g., 2 to 100, preferably 2 to 50, more preferably 2 to 20, even more preferably 2 to 10, still more preferably 2 to 5, and particularly preferably 2) amino acids are substituted, deleted, added, or inserted (preferably conservatively substituted) in the amino acid sequence of a wild-type double-strand break-generating Cas protein or a Nickase Cas protein based on the wild-type double-strand break-generating Cas protein, and having the activity thereof (the activity to bind to and cleave a target site of genomic DNA while forming a complex with guide RNA). The above "activity" can be evaluated in vitro or in vivo according to or in accordance with known methods.

The concatenated sequence b2 is not particularly limited as long as it is a sequence in which multiple (e.g., 2 to 6, 2 to 5, 2 to 4, 2 to 3, 2) identical or different nuclear localization signal sequences are linked together. The nuclear localization signal sequence is as described above in terms of the "nuclear localization signal" in the 5' to 3' exonuclease domain.

In the concatenated sequence b2, the nuclear localization signal sequences may be directly linked to each other not via an additional amino acid sequence or may be linked via an additional amino acid sequence (e.g., an amino acid sequence of about 1 to 50, 1 to 30, 1 to 20, 1 to 10, or 1 to 5 residues (e.g., a linker sequence)).

The concatenated sequence b2 preferably comprises both a monopartite nuclear localization signal sequence and a bipartite nuclear localization signal sequence, and more preferably comprises a combination of at least one monopartite nuclear localization signal sequence and a single bipartite nuclear localization signal sequence (a concatenated sequence of at least two nuclear localization signal sequences) (specifically, for example, SEQ ID NO: 91, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, and SEQ ID NO: 100). When both a monopartite nuclear localization signal sequence and a bipartite nuclear localization signal sequence are present, the bipartite nuclear localization signal sequence is preferably located on the N-terminal side of the monopartite nuclear localization signal sequence. Further, when both a monopartite nuclear localization signal sequence and a bipartite nuclear localization signal sequence are present, it is preferred that the bipartite nuclear localization signal sequence is located closer to the amino acid sequence b1 and that the monopartite nuclear localization signal sequence is located farther from the amino acid sequence b1 (i.e., it is preferred that the amino acid sequence b1, the bipartite nuclear localization signal sequence, and the monopartite nuclear localization signal sequence are arranged in this order from the N-terminal side, or that the monopartite nuclear localization signal sequence, the bipartite nuclear localization signal sequence, and the amino acid sequence b1 are arranged in this order from the N-terminal side).

In the MAD7 domain, the linkage mode between the amino acid sequence b1 and the concatenated sequence b2 is not particularly limited and may be directly linked to each other not via an additional amino acid sequence or may be linked via an additional amino acid sequence (e.g., an amino acid sequence of about 1 to 50, 1 to 30, 1 to 20, 1 to 10, or 1 to 5 residues (e.g., a linker sequence)).

The positional relationship between the amino acid sequence b1 and the concatenated sequence b2 in the MAD7 domain is not particularly limited, and includes both an embodiment in which the concatenated sequence b2 is located on the C-terminal side of the amino acid sequence b1 and an embodiment in which the concatenated sequence b2 is located on the N-terminal side of the amino acid sequence b1. In one embodiment of the present invention, the concatenated sequence b2 is preferably located on the C-terminal side of the amino acid sequence b1. In one embodiment of the present invention, when the 5' to 3' exonuclease domain is located on the N-terminal side of the MAD7 domain, the concatenated sequence b2 is preferably located on the C-terminal side of the amino acid sequence b1. On the other hand, when the 5' to 3' exonuclease domain is located on the C-terminal side of the MAD7 domain, the concatenated sequence b2 is preferably located on the N-terminal side of the amino acid sequence b1.

In Protein 1 of the present invention, the linkage mode between the 5' to 3' exonuclease domain and the MAD7 domain is not particularly limited and may be directly linked to each other not via an additional amino acid sequence or may be linked via an additional amino acid sequence (e.g., an amino acid sequence of about 1 to 50, 1 to 30, 1 to 20, 1 to 10, or 1 to 5 residues (e.g., a linker sequence)).

The positional relationship between the 5' to 3' exonuclease domain and the MAD7 domain in Protein 1 of the present invention is not particularly limited, and includes both an embodiment in which the 5' to 3' exonuclease domain is located on the N-terminal side of the MAD7 domain and an embodiment in which the 5' to 3' exonuclease domain is located on the C-terminal side of the MAD7 domain. In one embodiment of the present invention, the 5' to 3' exonuclease domain is preferably located on the N-terminal side of the MAD7 domain.

In a particularly preferred embodiment of Protein 1 of the present invention, the nuclear localization signal sequence a2, the amino acid sequence a1, the amino acid sequence b1, and the concatenated sequence b2 are arranged in this order from the N-terminal side.

In one embodiment of the present invention, as an embodiment different from those of Protein 1 of the present invention, the present invention relates to a protein comprising an amino acid sequence b1, wherein the amino acid sequence b1 is a mutant sequence of the amino acid sequence represented by SEQ ID NO: 6 and comprises at least one mutation selected from the group consisting of A24E, K169R, I180K, A290R, S469K, S469R, K535R, N583K, N583R, K590R, I646K, I646R, T714K, T714R, N827D, Y832K, Y832R, K970R, H1025K, H1025R, S1175A, and C1219N in the amino acid sequence represented by SEQ ID NO: 6 (which may be referred to herein as "Protein 2 of the present invention"). Further, in one embodiment of the present invention, as an embodiment different from those of Proteins 1 and 2 of the present invention, the present invention relates to a protein comprising (ai) a 5' to 3' exonuclease domain comprising an amino acid sequence a1 of 5' to 3' exonuclease and a nuclear localization signal sequence a2, and (bi) a MAD7 domain comprising an amino acid sequence b1 of MAD7 protein (which may be referred to herein as "Protein 3 of the present invention"). Below, Proteins 1 to 3 of the present invention may be collectively referred to as "the protein of the present invention."

In a preferred embodiment of Proteins 2 and 3 of the present invention, each configuration of Protein 1 of the present invention may be independently incorporated as a configuration of Proteins 2 and 3 of the present invention.

In a particularly preferred embodiment of Proteins 2 and 3 of the present invention, from the perspective of genome editing efficiency (in particular, genome editing efficiency in plant cells), the amino acid sequence b1 is preferably a mutant sequence of the amino acid sequence represented by SEQ ID NO: 6 and comprises a K169R mutation in the amino acid sequence represented by SEQ ID NO: 6, and more preferably further comprises, in addition to the K169R mutation, at least one mutation selected from the group consisting of S469R and H1025K.

In the above particularly preferred embodiment, it is further preferable to comprise (b) a MAD7 domain comprising the amino acid sequence b1 of MAD7 protein and a concatenated sequence b2 of multiple nuclear localization signal sequences.

The protein of the present invention may additionally comprise other amino acid sequences of proteins or peptides, such as protein tags, fluorescent proteins, and luminescent proteins, other than the 5' to 3' exonuclease domain and the MAD7 domain as long as genome editing efficiency is not significantly impaired. Examples of protein tags include His tag, FLAG tag, Halo tag, MBP tag, HA tag, Myc tag, V5 tag, PA tag, Sun tag, and the like.

The protein of the present invention may be a chemically modified protein as long as genome editing efficiency is not significantly impaired.

The C-terminus of the protein of the present invention may be a carboxyl group (-COOH), carboxylate (-COO⁻), amide (-CONH₂), or ester (-COOR).

Examples of R in the ester include C₁₋₆ alkyl groups, such as methyl, ethyl, n-propyl, isopropyl, and n-butyl; C₃₋₈ cycloalkyl groups, such as cyclopentyl and cyclohexyl; C₆₋₁₂ aryl groups, such as phenyl and α-naphthyl; phenyl-C₁₋₂ alkyl groups, such as benzyl and phenethyl; C₇₋₁₄ aralkyl groups, such as α-naphthyl-C₁₋₂ alkyl groups, such as α-naphthylmethyl; a pivaloyloxymethyl group; and the like.

In the protein of the present invention, a carboxyl group (or carboxylate) other than that at the C-terminus may be amidated or esterified. Examples of esters for use in this case include the esters at the C-terminus listed above.

In addition, the protein of the present invention also encompasses, for example, proteins in which the amino group of the amino acid residue at the N-terminus is protected by a protecting group (e.g., a C₁₋₆ acyl group, such as a formyl group, an acetyl group, or another C₁₋₆ alkanoyl group); proteins in which a glutamine residue at the N-terminus is pyroglutamated, which can be generated as a result of in vivo cleavage; proteins in which a substituent (e.g., -OH, -SH, an amino group, an imidazole group, an indole group, and a guanidino group) on a side chain of an amino acid in the molecule is protected by an appropriate protecting group (e.g., a C₁₋₆ acyl group, such as a formyl group, an acetyl group, or another C₁₋₆ alkanoyl group); and complex proteins, such as "glycoproteins," in which sugar chains are bonded.

The protein of the present invention may be in the form of a salt with an acid or base. The salt is not particularly limited and may be an acidic salt or a basic salt. Examples of acidic salts include inorganic acid salts, such as hydrochloride, hydrobromide, sulfate, nitrate, and phosphate; organic acid salts, such as acetate, propionate, tartrate, fumarate, maleate, malate, citrate, methanesulfonate, and paratoluenesulfonate; amino acid salts, such as aspartate and glutamate; and the like. Examples of basic salts include alkali metal salts, such as sodium and potassium salts; alkaline earth metal salts, such as calcium and magnesium salts; and the like.

The protein of the present invention may be in the form of a solvate. Examples of solvents include, but are not particularly limited to, water, ethanol, glycerol, acetic acid, and the like.

The protein of the present invention can be easily produced according to known genetic engineering techniques. For example, the protein of the present invention can be produced using PCR, restriction enzyme cleavage, DNA ligation techniques, in vitro transcription and translation techniques, recombinant protein production techniques, and the like. Examples of recombinant protein production techniques include methods of using cultured cells as hosts, methods of using plants, such as tobacco, as hosts, and the like.

### 3. Polynucleotide

In one embodiment of the present invention, the present invention relates to a polynucleotide comprising a coding sequence for the protein of the present invention (which may be referred to herein as "the polynucleotide of the present invention"), and to a cell comprising the polynucleotide of the present invention (which may be referred to herein as "the cell of the present invention"). These are described below.

The coding sequence for the protein of the present invention is not particularly limited as long as it is a polynucleotide comprising the base sequence encoding the protein of the present invention.

In one embodiment of the present invention, the polynucleotide of the present invention comprises an expression cassette of the protein of the present invention.

The expression cassette of the protein of the present invention is not particularly limited as long as it is a polynucleotide capable of expressing the protein of the present invention in cells. A typical example of the expression cassette of the protein of the present invention includes a polynucleotide comprising a promoter and the coding sequence for the protein of the present invention placed under the control of the promoter.

The promoter in the expression cassette of the protein of the present invention is not particularly limited and can be suitably selected according to the target cell. The promoters for use can be, for example, various pol II promoters. Examples of pol II promoters include, but are not particularly limited to, CMV promoter, EF1 promoter, SV40 promoter, MSCV promoter, and the like. Other examples of promoters include RPS5A promoter, UBQ promoter, CaMV35S promoter, NOS promoter, CmYLCV promoter, tryptophan promoters, such as trc and tac, lac promoter, T7 promoter, T5 promoter, T3 promoter, SP6 promoter, arabinose-inducible promoter, cold-shock promoter, tetracycline-inducible promoter, and the like.

The expression cassette of the protein of the present invention may optionally comprise other elements (e.g., multicloning sites (MCS), replication origins, enhancer sequences, repressor sequences, insulator sequences, terminator sequences, reporter protein (e.g., fluorescent protein)-coding sequences, drug-resistance gene-coding sequences, and guide RNA expression cassettes).

The polynucleotide of the present invention can be in the form of a vector. A suitable vector is selected according to the purpose of use (cloning, protein expression) and in consideration of the type of host cells. Examples of vectors that use *Escherichia coli* as the host include M13 phage or its variants, λ phage or its variants, pBR322 or its variants (e.g., pB325, pAT153, and pUC8), and the like. Examples of vectors that use yeast as the host include pYepSec1, pMFa, pYES2, pPIC3.5K, and the like. Examples of vectors that use an insect cell as the host include pAc, pVL, and the like. Examples of vectors that use a mammalian cell as the host include pcDNA, pCDM8, pMT2PC, and the like. Other examples include viral vectors, such as retrovirus, lentivirus, adenovirus, adeno-associated virus, herpes virus, Sendai virus, tobacco mosaic virus, cucumber mosaic virus, African cassava mosaic virus, apple latent spherical virus, barley stripe mosaic virus, bean pod mottle virus, beet curly top virus, brome mosaic virus, cabbage leaf curl virus, cotton leaf crumple virus, cymbidium mosaic virus, grapevine virus A, pea early browning virus, poplar mosaic virus, potato virus X, rice tungro bacilliform virus, satellite tobacco mosaic virus, tobacco curly shoot virus, tobacco rattle virus, and bean yellow dwarf virus.

In one embodiment of the present invention, the present invention relates to a polynucleotide comprising (aii) an expression cassette of a protein comprising a 5' to 3' exonuclease domain comprising an amino acid sequence a1 of 5' to 3' exonuclease and a nuclear localization signal sequence a2, and (bii) an expression cassette of a protein comprising a MAD7 domain comprising an amino acid sequence b1 of MAD7 protein and a concatenated sequence b2 of multiple nuclear localization signal sequences. This polynucleotide is also encompassed by "the polynucleotide of the present invention." Each configuration of the polynucleotide is the same as those described above. The polynucleotide may be a polynucleotide that comprises both the expression cassette (aii) and the expression cassette (bii) in the same molecule, or may be a combination of a polynucleotide that comprises the expression cassette (aii) and a polynucleotide that is a different molecule and comprises the expression cassette (bii).

The cell of the present invention is not particularly limited as long as it comprises the polynucleotide of the present invention. Examples of the cells include Escherichia coli, such as *Escherichia coli* K12; *Bacillus* bacteria, such as *Bacillus subtilis* MI114; yeast, such as *Saccharomyces cerevisiae* AH22, Sf cell line from *Spodoptera frugiperda* or HighFive cell line from *Trichoplusia ni;* insect cells, such as olfactory nerve cells; animal cells, such as COS7 cells; plant cells; and the like. Preferred examples of animal cells include mammalian-derived cultured cells, and specific examples include COS7 cells, CHO cells, HEK293 cells, HEK293FT cells, Hela cells, PC12 cells, N1E-115 cells, SH-SY5Y cells, and the like.

### 4. Composition, Kit, Genome Editing Method, and Production Method

The polynucleotide of the present invention and the protein of the present invention can be used as a composition for genome editing or as a kit for genome editing. The present invention can provide a genome editing method and a method for producing genome-edited matter (a cell or organism) by using the polynucleotide of the present invention.

The composition for genome editing is not particularly limited as long as it comprises at least one member selected from the group consisting of the protein of the present invention and the polynucleotide of the present invention and may further optionally comprise other components. Examples of other components include, but are not particularly limited to, bases, carriers, solvents, dispersants, emulsifiers, buffers, stabilizers, excipients, binders, disintegrants, lubricants, thickeners, moisturizers, colorants, flavoring agents, chelating agents, and the like. The composition for genome editing may optionally comprise a polynucleotide comprising a guide RNA expression cassette. A donor polynucleotide may also be contained, if necessary.

The kit for genome editing is not particularly limited as long as it comprises at least one member selected from the group consisting of the protein of the present invention and the polynucleotide of the present invention, and may optionally suitably comprise other materials, reagents, instruments, etc. that are necessary for the implementation of the genome editing method of the present invention, such as nucleic acid introduction reagents and buffer solutions. As other materials necessary for the implementation of the genome editing method of the present invention, the kit for genome editing may optionally comprise a polynucleotide comprising a guide RNA expression cassette. A donor polynucleotide may also be contained, if necessary.

The genome editing method and the method for producing genome-edited matter (a cell or organism) comprise introducing at least one member selected from the group consisting of the protein of the present invention and the polynucleotide of the present invention into a cell or organism.

The target cells and organisms for genome editing are not particularly limited as long as they are cells and organisms that can be subjected to genome editing by the CRISPR/Cas system. The target cells for genome editing may be cells from various tissue sources or cells with various properties. Examples include blood cells, hematopoietic stem/progenitor cells, gametes (sperm and egg), fertilized eggs, fibroblasts, epithelial cells, vascular endothelial cells, nerve cells, hepatocytes, keratin-producing cells, muscle cells, epidermal cells, endocrine cells, ES cells, iPS cells, tissue stem cells, cancer cells, leaf cells, pollen, shoot apical cells, and the like. Examples of the target organisms for genome editing include mammalian animals, such as humans, monkeys, mice, rats, dogs, cats, and rabbits; amphibian animals, such as African clawed frogs; fish animals, such as zebrafish, killifish, and tiger pufferfish; chordates, such as ascidians; arthropods, such as Drosophilidae and silkworms; fungi, such as yeast and *Neurospora crassa;* bacteria, such as *Escherichia coli, Bacillus subtilis,* and blue-green algae; and the like. Examples of the target organisms for genome editing also include plants. Examples of plants include a wide range of plants, including bryophytes, pteridophytes, gymnosperms, magnoliids of angiosperms, monocots, and eudicots (eurosid I, eurosid II, euasterids I, euasterids II, and their out-groups). More specific examples of plants include *Solanaceaeplants,* such as tomatoes, green bell peppers, *Capsicum annuum,* eggplants, *Nicotiana tabacum,* and *Solanum torvum; Cucurbitaceae* plants, such as cucumber, *Cucurbita* plants, melon, and watermelon; *Brassicaceaeplants,* such as cabbage, broccoli, Chinese cabbage, and mustard; green leaf and spicy leaf vegetables, such as celery, parsley, and lettuce; *Liliaceaeplants,* such as Welsh onions, onions, and garlic; other fruit and vegetable crops, such as strawberries; taproot plants, such as *daikon* radish, white turnip, carrot, and greater burdock; roots and tubers, such as taros, cassava, potatoes, sweet potatoes, and Chinese yam; grains, such as rice, corn, wheat, sorghum, barley, rye, purple false brome, and buckwheat; beans, such as soybeans, adzuki beans, mung beans, black-eyed beans, *Phaseolus vulgaris,* peanuts, peas, and *Vicia faba;* other vegetables, such as asparagus, spinach, and *Cryptotaenia japonica;* flower plants, such as *Eustoma grandiflorum, Matthiola incana, Dianthus caryophyllus,* and *Chrysanthemum morifolium;* grass, such as *Agrostis stolonifera* and *Zoysia matrella;* oil crops, such as rapeseed, peanuts, *Brassica napus, Jatropha curcas,* sesame, and *Perilla frutescens;* fiber crops, such as cotton and *Juncus effusus;* feed crops, such as clover, dent corn, and *Medicago truncatula;* deciduous fruit trees, such as apples, pears, grapes, and peaches; citrus fruits, such as *Citrus unshiu,* oranges, lemons, and grapefruits; woody plants, such as *Rhododendron indicum,* Rhododendron plants, *Cryptomeria, Populus,* and *Hevea brasiliensis;* and the like.

The introduction method is not particularly limited and can be suitably selected according to the type of the target cell or organism and the type of the substance to be introduced (e.g., whether it is a nucleic acid or a protein). Examples of the introduction method include microinjection, electroporation, DEAE-dextran treatment, lipofection, nanoparticle-mediated transfection, virus-mediated nucleic acid delivery, and the like. If the target is a plant, examples of the introduction method include Agrobacterium methods, such as floral dip and floral spray methods; particle gun methods; virus-mediated nucleic acid delivery; and the like.

Genome editing occurs in the target cells or organisms after a specific period of time has elapsed since the introduction; thus, by collecting those cells or organisms, genome-edited cells or organisms can be obtained.

The presence of genome editing can be confirmed by using a nucleic acid obtained from the target product and following known methods, such as T7 Endonuclease I assay, CAPS (cleaved amplified polymorphic sequence), and a method for detecting a mutation in the base sequence around the target site for genome editing (around the cleavage site of target-specific nuclease) (e.g., a PCR method that uses primers designed for the predicted mutation site and subsequent sequencing analysis).

### Examples

The present invention is described in detail below based on Examples; however, the present invention is not limited to these Examples.

### Production Example 1: Vector Construction

The following reagents and outsourcing services were used for vector cloning unless otherwise noted. PrimeSTAR Max (Takara Bio Inc.) was used for PCR amplification. The NucleoSpin Gel and PCR Clean-up kit (Macherey-Nagel) were used to purify PCR products. For the Golden Gate method, the NEB Golden Gate Assembly Kit (BsaI-HF v2) (New England Biolabs Japan, Inc.) was used. For cloning an insert into the pMiniT 2.0 vector, the NEB PCR Cloning Kit (New England BioLabs Japan, Inc.) was used. The 2x GeneArt Enzyme Mix (Thermo Fisher Scientific K.K.) was used for assembly (seamless cloning) between DNA fragments (vector and insert) with homologous sequences at the ends. Sequence analysis (Sanger method) and synthesis of oligonucleotides (primers) were outsourced to Eurofins Genomics K.K.

### Production Example 1-1: Production of Reporter Vector

Reporter vectors were produced for use in the SSA assay of Test Example 1 (an assay to measure genome editing efficiency) described below. Fig. 1 shows the overview of the assay. A reporter gene as is in a reporter vector is a gene that expresses a protein without reporter activity (inactive reporter gene); however, when a double-strand break occurs in an inactive reporter gene by genome editing, annealing occurs via the overlapped regions, and the gene is changed into a gene that expresses a protein with reporter activity (active reporter gene). Therefore, genome editing efficiency can be determined by measuring the activity of the reporter protein expressed from the active reporter gene.

The base sequence of the reporter gene was designed by modifying the cloning site of pGL4-SSA, which is a material described in Sakuma et al. (2013), Efficient TALEN construction and evaluation methods for human cell and animal applications, Genes to Cells, Vol. 18 (Issue 4), pp. 315-326, to contain two BpiI sites. Additionally, BsaI restriction enzyme sites were specified to be added to both ends of the DNA fragment. A pUC57-pSSA vector, in which the DNA fragment above was integrated, was obtained by outsourcing the production (artificial synthesis) to GenScript Japan Inc.

The target sequence was set to a sequence from the *Nicotiana benthamiana* PDS gene. To insert the target sequence into the vector, an oligonucleotide comprising the target sequence from the *Nicotiana benthamiana* PDS gene and an oligonucleotide comprising a sequence complementary to the target sequence were prepared. The two oligonucleotides comprising the target sequence were then annealed, and the annealed oligonucleotides were inserted into the synthetic vector above by using treatment with a BpiI restriction enzyme (Thermo Fisher Scientific K.K.) and a ligation reaction to obtain a pUC57-pSSA-NbPDS vector. In this vector, the target sequence was located at the site indicated by "∇" in Fig. 1.

A pALTER-MAX vector (Promega K.K.) was prepared as a vector for gene expression in mammalian cells, and the multicloning site was modified by a seamless cloning method to allow cloning with a BsaI restriction enzyme to thus obtain a pALTER-MAX-GG vector. Then, the reporter gene portion pSSA-NbPDS in the pUC57-pSSA-NbPDS vector was inserted into the pALTER-MAX-GG vector by using the Golden Gate cloning method to construct a luciferase reporter vector pALTER-pSSA-NbPDS, which comprised the target sequence. At the same time, a luciferase reporter vector pALTER-pSSA, which did not comprise the target sequence, was constructed as a negative control for genome editing detection using pUC57-pSSA, which did not comprise the target sequence.

Produced Vector: reporter vector (pALTER vector)
Entry vector
   - pUC57-pSSA
   - pUC57-pSSA-NbPDS
   - pALTER-MAX-GG
Mammalian cell expression vector
   - pALTER-pSSA-NbPDS
   - pALTER-pSSA

### Production Example 1-2: Production of Guide RNA Expression Vector

For the production of guide RNA expression vectors, a pEXA2J2-hU6-crRNA-NbPDS vector into which a DNA fragment comprising the human U6 promoter sequence, a direct repeat sequence, a sequence from *Nicotiana benthamiana* PDS gene as a spacer, and a poly T sequence was integrated, was obtained by outsourcing the production (artificial synthesis) to Eurofins Genomics K.K. Subsequently, the direct repeat sequence portion was modified by seamless cloning to obtain two types of crRNA expression vectors (pEXA2J2-hU6-crRNAqa2-NbPDS and pEXA2J2-hU6-crRNAqa2GT-NbPDS). The pEXA2J2-hU6-crRNAqa2-NbPDS vector was first produced by using the synthetic vector mentioned above as a template plasmid, and the produced vector was then used as a template plasmid to obtain pEXA2J2-hU6-crRNAqa2GT-NbPDS. More specifically, PCR was first performed using a mutation-introducing primer pair and the template plasmid, and after digestion of template DNA in the reaction liquid, the PCR product was purified on a column. The purified PCR product was mixed with 2x GeneArt Enzyme Mix in equal amounts and allowed to incubate at room temperature for 30 minutes. Subsequently, the total amount of the reaction liquid obtained above and competent cells (TOP10, Thermo Fisher Scientific K.K.) were used for transformation of *Escherichia coli* by a common heat-shock method. Thereafter, the plasmid was extracted from the transformed *Escherichia coli* with Wizard Plus SV Minipreps DNA Purification Systems (Promega K.K.), and the base sequence was confirmed by sequence analysis (Sanger method). The primer sequences used to modify the direct repeat sequence portion and the name of the modified vectors are as follows.

Produced Vector: guide RNA expression vector
- pEXA2J2-hU6-crRNA-NbPDS
- pEXA2J2-hU6-crRNAqa2-NbPDS (sequences of primers used in the production: SEQ ID NOs: 1 and 2)
- pEXA2J2-hU6-crRNAqa2GT-NbPDS (sequences of primers used in the production: SEQ ID NOs: 3 and 4)

### Production Example 1-3: Production of Effector Expression Vector: MAD7 Gene Expression Vector

A pEXA2J2-3xFLAG-NLS-MAD7-NLS vector, in which a DNA fragment obtained by in-frame fusion of a 3xFLAG epitope tag (base sequence: SEQ ID NO: 7, amino acid sequence: SEQ ID NO: 8) and a SV40 nuclear localization signal (base sequence: SEQ ID NO: 9, amino acid sequence: SEQ ID NO: 10) to the immediate downstream of the start codon in a human codon-optimized MAD7 gene (base sequence: SEQ ID NO: 5, amino acid sequence: SEQ ID NO: 6) (with a spacer (6 bases long, 2 amino acid residues) being located between 3xFLAG and SV40 NLS, and a spacer (24 bases long, 8 amino acid residue) being located between SV40 NLS and MAD7), and a nucleoplasmin nuclear localization signal (base sequence: SEQ ID NO: 11, amino acid sequence: SEQ ID NO: 12) to the C-terminus (immediately upstream of the stop codon) was integrated, was obtained by outsourcing the production (artificial synthesis) to Eurofins Genomics K.K. After BsaI restriction enzyme sites were added to both ends of the coding region portion, the vector was cloned into a pMiniT2.0 vector (New England Biolabs Japan Inc.) to obtain a pMT-3xFLAG-NLS-MAD7-NLS vector.

For gene expression in mammalian cells, the gene portion 3xFLAG-NLS-MAD7-NLS in the pMT-3xFLAG-NLS-MAD7-NLS vector was inserted into the pALTER-MAX-GG vector by using the Golden Gate cloning method to construct pALTER-3xFLAG-NLS-MAD7-NLS.

Produced Vector: effector expression vector (pALTER vector)
Entry vector
   - pMT-3xFLAG-NLS-MAD7-NLS
Mammalian cell expression vector
   - pALTER-3xFLAG-NLS-MAD7-NLS

### Production Example 1-4: Production of Effector Expression Vector: MAD7 Expression Vector in Which the Location of Nuclear Localization Signal (NLS) Was Modified

First, a pMT-MAD7-NLS vector in which the 3xFLAG epitope tag and SV40 nuclear localization signal present on the N-terminal side of the pMT-3xFLAG-NLS-MAD7-NLS vector were deleted, and a pMT-NLS-MAD7-NLS vector in which only the 3xFLAG epitope tag was deleted were produced. The deletion was performed by PCR using mutation-introducing primer pairs and the pMT-3xFLAG-NLS-MAD7-NLS vector as a incubate plasmid in the same manner as in the mutation-introducing method described in Production Example 1-2. The primer sequences used for the deletion and the names of the vectors after introducing the deletion are shown below in the "Produced Vector" section.

Additionally, a PEXK4J2_2xNLS vector, in which a DNA fragment obtained by fusion of a nucleoplasmin nuclear localization signal (base sequence: SEQ ID NO: 11, amino acid sequence: SEQ ID NO: 12), a linker (6 bases long, 2 amino acid residues), a 3xHA epitope tag (base sequence: SEQ ID NO: 13, amino acid sequence: SEQ ID NO: 14), a linker (6 bases long, 2 amino acid residues), and a SV40 nuclear localization signal (base sequence: SEQ ID NO: 9, amino acid sequence: SEQ ID NO: 10) in this order was integrated, was obtained by outsourcing the production (artificial synthesis) to Eurofins Genomics K.K. A DNA fragment (insert) was obtained by PCR amplification of the region containing two NLSs from the artificially synthesized vector, and the DNA sequence encoding the one nucleoplasmin nuclear localization signal present in the C-terminus region of the MAD7 gene on the pMT-MAD7-NLS vector was replaced with this DNA fragment to obtain a pMT-MAD7-2xNLS vector. More specifically, the vector was constructed by separately preparing two DNA fragments with homologous sequences at the ends (vector and insert) by PCR amplification and seamlessly assembling the homologous sequences.

The insertion into the pALTER-MAX-GG vector for gene expression in mammalian cells was performed in the same manner as described in Production Example 1-3.

Produced Vector: effector expression vector (pALTER vector)
Entry vector
   - pMT-MAD7-NLS (sequences of primers used in the production: SEQ ID NOs: 15 and 16)
   - pMT-NLS-MAD7-NLS (sequences of primers used in the production: SEQ ID NOs: 17 and 18)
   - pMT-MAD7-2xNL5
Mammalian cell expression vector
   - pALTER-MAD7-NLS
   - pALTER-NLS-MAD7-NLS
   - pALTER-MAD7-2xNLS

### Production Example 1-5: Effector Expression Vector: MAD7 Expression Vector in Which Amino Acid Mutation Was Introduced

An amino acid mutation of K169R, K535R, or S1175A was introduced into MAD7 (amino acid sequence: SEQ ID NO: 6) in the same manner as described in Production Example 1-2 by using mutation-introducing primer pairs and the pMT-3xFLAG-NLS-MAD7-NLS vector as a template plasmid to obtain a pMT-3xFLAG-NLS-MAD7-NLS-[mutation name] vector. Then, deletion of the 3xFLAG epitope tag and the SV40 nuclear localization signal from the N-terminus and insertion of 2xNLS into the C-terminus were performed according to the same procedure as described in Production Example 1-4 to obtain a pMT-MAD7-2xNLS-[mutation name] vector.

The other mutations were introduced in the same manner as described in Production Example 1-2 by using mutation-introducing primer pairs and the pMT-MAD7-2xNLS vector as a template plasmid to produce a pMT-MAD7-2xNLS-[mutation name] vector. The primer sequences used for mutation introduction and the names of the vectors after mutation introduction are shown below in the "Produced Vector" section.

The insertion into the pALTER-MAX-GG vector for gene expression in mammalian cells was performed in the same manner as described in Production Example 1-3.

Produced Vector: effector expression vector (pALTER vector)
Entry vector
   - pMT-MAD7-2xNL5-A24E (sequences of primers used in the production: SEQ ID NOs: 19 and 20)
   - pMT-MAD7-2xNLS-K169R (sequences of primers used in the production: SEQ ID NOs: 21 and 22)
   - pMT-MAD7-2xNLS-I180K (sequences of primers used in the production: SEQ ID NOs: 23 and 24)
   - pMT-MAD7-2xNLS-A290R (sequences of primers used in the production: SEQ ID NOs: 25 and 26)
   - pMT-MAD7-2xNL5-5469K (sequences of primers used in the production: SEQ ID NOs: 27 and 28)
   - pMT-MAD7-2xNL5-5469R (sequences of primers used in the production: SEQ ID NOs: 29 and 30)
   - pMT-MAD7-2xNLS-K535R (sequences of primers used in the production: SEQ ID NOs: 31 and 32)
   - pMT-MAD7-2xNLS-N583K (sequences of primers used in the production: SEQ ID NOs: 33 and 34)
   - pMT-MAD7-2xNLS-N583R (sequences of primers used in the production: SEQ ID NOs: 35 and 36)
   - pMT-MAD7-2xNLS-K590R (sequences of primers used in the production: SEQ ID NOs: 37 and 38)
   - pMT-MAD7-2xNL5-I646K (sequences of primers used in the production: SEQ ID NOs: 39 and 40)
   - pMT-MAD7-2xNL5-I646R (sequences of primers used in the production: SEQ ID NOs: 41 and 42)
   - pMT-MAD7-2xNLS-T714K (sequences of primers used in the production: SEQ ID NOs: 43 and 44)
   - pMT-MAD7-2xNLS-T714R (sequences of primers used in the production: SEQ ID NOs: 45 and 46)
   - pMT-MAD7-2xNL5-N827D (sequences of primers used in the production: SEQ ID NOs: 47 and 48)
   - pMT-MAD7-2xNLS-Y832K (sequences of primers used in the production: SEQ ID NOs: 49 and 50)
   - pMT-MAD7-2xNL5-Y832R (sequences of primers used in the production: SEQ ID NOs: 51 and 52)
   - pMT-MAD7-2xNLS-K970R (sequences of primers used in the production: SEQ ID NOs: 53 and 54)
   - pMT-MAD7-2xNLS-H1025K (sequences of primers used in the production: SEQ ID NOs: 55 and 56)
   - pMT-MAD7-2xNLS-H1025R (sequences of primers used in the production: SEQ ID NOs: 57 and 58)
   - pMT-MAD7-2xNLS-S1175A (sequences of primers used in the production: SEQ ID NOs: 59 and 60)
   - pMT-MAD7-2xNLS-C1219N (sequences of primers used in the production: SEQ ID NOs: 61 and 62)
   - pMT-3xFLAG-NLS-MAD7-NLS-K169R
   - pMT-3xFLAG-NLS-MAD7-NLS-K535R
   - pMT-3xFLAG-NLS-MAD7-NLS-S1175A
Mammalian cell expression vector
   - pALTER-MAD7-2xNL5-A24E
   - pALTER-MAD7-2xNLS-K169R
   - pALTER-MAD7-2xNLS-I180K
   - pALTER-MAD7-2xNL5-A290R
   - pALTER-MAD7-2xNL5-5469K
   - pALTER-MAD7-2xNL5-5469R
   - pALTER-MAD7-2xNLS-K535R
   - pALTER-MAD7-2xNLS-N583K
   - pALTER-MAD7-2xNL5-N583R
   - pALTER-MAD7-2xNL5-K590R
   - pALTER-MAD7-2xNL5-I646K
   - pALTER-MAD7-2xNL5-I646R
   - pALTER-MAD7-2xNLS-T714K
   - pALTER-MAD7-2xNL5-T714R
   - pALTER-MAD7-2xNL5-N827D
   - pALTER-MAD7-2xNL5-Y832K
   - pALTER-MAD7-2xNL5-Y832R
   - pALTER-MAD7-2xNLS-K970R
   - pALTER-MAD7-2xNLS-H1025K
   - pALTER-MAD7-2xNLS-H1025R
   - pALTER-MAD7-2xNLS-S1175A
   - pALTER-MAD7-2xNLS-C1219N

### Production Example 1-6: Effector Expression Vector: MAD7 Expression Vector in Which Multiple Amino Acid Mutations Were Introduced

Mutation introduction was performed in the same manner as described in Production Example 1-2 by using mutation-introducing primer pairs and the pMT-MAD7-2xNLS-K169R vector as a template plasmid to obtain a pMT-MAD7-2xNLS-[mutation name] vector. The primer sequences used for mutation introduction and the names of the vectors after mutation introduction are shown below in the "Produced Vector" section.

The insertion into the pALTER-MAX-GG vector for gene expression in mammalian cells was performed in the same manner as described in Production Example 1-3.
Produced Vector: effector expression vector (pALTER vector)
Entry vector
   - pMT-MAD7-2xNLS-K169R/S469K (sequences of primers used in the production: SEQ ID NOs: 27 and 28)
   - pMT-MAD7-2xNLS-K169R/S469R (sequences of primers used in the production: SEQ ID NOs: 29 and 30)
   - pMT-MAD7-2xNLS-K169R/N583K (sequences of primers used in the production: SEQ ID NOs: 33 and 34)
   - pMT-MAD7-2xNLS-K169R/N583R (sequences of primers used in the production: SEQ ID NOs: 35 and 36)
   - pMT-MAD7-2xNLS-K169R/Y832K (sequences of primers used in the production: SEQ ID NOs: 49 and 50)
   - pMT-MAD7-2xNLS-K169R/Y832R (sequences of primers used in the production: SEQ ID NOs: 51 and 52)
   - pMT-MAD7-2xNL5-K169R/H1025K (sequences of primers used in the production: SEQ ID NOs: 55 and 56)
   - pMT-MAD7-2xNLS-K169R/H1025R (sequences of primers used in the production: SEQ ID NOs: 57 and 58)
Mammalian cell expression vector
   - pALTER-MAD7-2xNLS-K169R/S469K
   - pALTER-MAD7-2xNLS-K169R/S469R
   - pALTER-MAD7-2xNLS-K169R/N583K
   - pALTER-MAD7-2xNLS-K169R/N583R
   - pALTER-MAD7-2xNLS-K169R/Y832K
   - pALTER-MAD7-2xNLS-K169R/Y832R
   - pALTER-MAD7-2xNLS-K169R/H1025K
   - pALTER-MAD7-2xNLS-K169R/H1025R

### Production Example 1-7: Production of Effector Expression Vector: MAD7 Expression Vector in Which T5 Exonuclease Was Fused to the N-terminal Side

A pEXK4J2_T5 vector, in which the D15 gene encoding a phage-derived T5 exonuclease (base sequence: SEQ ID NO: 63, amino acid sequence: SEQ ID NO: 64) and the DNA sequence encoding an XTEN peptide linker were integrated in this order while excluding the start codon (ATG), was obtained by outsourcing the production (artificial synthesis) to Eurofins Genomics K.K. A DNA fragment (insert) in which the D15 gene and the XTEN linker region were amplified by PCR from the synthetic vector was inserted into the immediate downstream of the SV40 nuclear localization signal on the pMT-3xFLAG-NLS-MAD7-NLS vector and into the immediate downstream of the start codon of the pMT-MAD7-2xNLS vector to obtain a PMT-3xFLAG-NLS-T5-MAD7-NLS vector (between 3xFLAG and NLS: a spacer (6 bases long, 2 amino acid residues), between NLS and T5: a spacer (24 bases long, 8 amino acid residues), and between T5 and MAD7: an XTEN linker (48 bases long, 16 amino acid residues), and a PMT-T5-MAD7-2xNLS vector (between T5 and MAD7: an XTEN linker (48 bases long, 16 amino acid residues). In addition, the 3xFLAG-NLS-T5- portion in the pMT-3xFLAG-NLS-T5-MAD7-NLS vector was amplified by PCR and inserted into the immediate downstream of the start codon of the pMT-MAD7-2xNLS vector to obtain a pMT-3xFLAG-NLS-T5-MAD7-2xNLS vector. More specifically, the procedure was performed by separately preparing two DNA fragments with homologous sequences at the ends (vector and insert) by PCR amplification and seamlessly assembling the homologous sequences.

The insertion into the pALTER-MAX-GG vector for gene expression in mammalian cells was performed in the same manner as described in Production Example 1-3.

Produced Vector: effector expression vector (pALTER vector)
Entry vector
   - pMT-3xFLAG-NLS-T5-MAD7-NLS
   - pMT-T5-MAD7-2xNLS
   - pMT-3xFLAG-NLS-T5-MAD7-2xNL5
Mammalian cell expression vector
   - pALTER-3xFLAG-NLS-T5-MAD7-NLS
   - pALTER-T5-MAD7-2xNLS
   - pALTER-3xFLAG-NLS-T5-MAD7-2xNLS

### Production Example 1-8: Effector Expression Vector: Amino Acid-mutated MAD7 Expression Vector Comprising Two NLSs at the C-terminus and a T5 Exonuclease Fused to the N-terminal Side

pMT-3xFLAG-NLS-MAD7-NLS-K169R/K535R was obtained by introducing a mutation in the same manner as described in Production Example 1-5 by using the pMT-3xFLAG-NLS-MAD7-NLS-K169R vector as a template plasmid. Then, a pMT-MAD7-2xNL5-K169R/K535R vector was obtained by deletion of the 3xFLAG epitope tag and the SV40 nuclear localization signal from the N-terminus and insertion of 2xNLS into the C-terminus according to the same procedure as described in Production Example 1-4. Further, pMT-MAD7-2xNLS-K169R/S1175A and pMT-MAD7-2xNLS-K169R/K535R/S1175A vectors were produced using the pMT-MAD7-2xNLS-K169R and pMT-MAD7-2xNL5-K169R/K535R vectors as template plasmids, respectively.

The 3xFLAG-NLS-T5 portion in the pMT-3xFLAG-NLS-T5-MAD7-NLS vector was amplified by PCR and inserted into the immediate downstream of the start codons of the pMT-MAD7-2xNLS-K169R, pMT-MAD7-2xNLS-K169R/K535R, pMT-MAD7-2xNLS-K169R/S1175A, and pMT-MAD7-2xNLS-K169R/K535R/S1175A vectors to respectively obtain PMT-3xFLAG-NLS-T5-MAD7-2xNLS-K169R, pMT-3xFLAG-NLS-T5-MAD7-2xNLS-K169R/K535R, pMT-3xFLAG-NLS-T5-MAD7-2xNL5-K169R/S1175A, and pMT-3xFLAG-NLS-T5-MAD7-2xNLS-K169R/K535R/S1175A vectors. More specifically, the procedure was performed by separately preparing two DNA fragments with homologous sequences at the ends (vector and insert) by PCR amplification and seamlessly assembling the homologous sequences.

The insertion into the pALTER-MAX-GG vector for gene expression in mammalian cells was also performed in the same manner as described in Production Example 1-3.

Produced Vector: effector expression vector (pALTER vector)
Entry vector
   - pMT-3xFLAG-NLS-MAD7-NLS-K169R/K535R
   - pMT-MAD7-2xNLS-K169R/K535R
   - pMT-MAD7-2xNLS-K169R/S1175A
   - pMT-MAD7-2xNLS-K169R/K535R/S1175A
   - pMT-3xFLAG-NLS-T5-MAD7-2xNLS-K169R
   - pMT-3xFLAG-NLS-T5-MAD7-2xNLS-K169R/K535R
   - pMT-3xFLAG-NLS-T5-MAD7-2xNLS-K169R/S1175A
   - pMT-3xFLAG-NLS-T5-MAD7-2xNLS-K169R/K535R/S1175A
Mammalian cell expression vector
   - pALTER-3xFLAG-NLS-T5-MAD7-2xNLS-K169R
   - pALTER-3xFLAG-NLS-T5-MAD7-2xNLS-K169R/K535R
   - pALTER-3xFLAG-NLS-T5-MAD7-2xNLS-K169R/S1175A
   - pALTER-3xFLAG-NLS-T5-MAD7-2xNLS-K169R/K535R/S1175A

### Production Example 2: Production of Effector Expression Vector in Plant

An effector expression vector was obtained by inserting a MAD7 protein expression cassette and a guide RNA expression cassette into a pTTK352 binary vector comprising the sequence of a geminivirus vector (bean yellow dwarf virus (BeYDV)).

The effector expression vector was produced as follows. An artificially synthesized, BeYDV-derived sequence was inserted by seamless cloning between the left and right borders of the pKIR vector (binary) described in a previous report (Tsutsui et al. (2016), pKAMA-ITACHI Vectors for Highly Efficient CRISPR/Cas9-Mediated Gene Knockout in Arabidopsis thaliana, Plant and Cell Physiology, Vol. 58 (Issue 1), pp. 46-56). MP and CP derived from BeYDV were deleted, and the sequence encoding a replicase protein, as well as the long intergenic region (LIR) and small intergenic region (SIR), were used.

The inserted MAD7 protein expression cassette was DNA in which the following items were arranged sequentially from the 5' end side:
(1) Cestrum yellow leaf curling virus (CmYLCV) promoter (SEQ ID NO: 80);
(2) dMac3 sequence (SEQ ID NO: 81);
(3) the MAD7 protein coding sequence below:
   a. MAD7-2xNLS,
   b. MAD7-2xNLS-K169R,
   c. MAD7-2xNLS-N583R,
   d. MAD7-2xNLS-K169R/S469R,
   e. MAD7-2xNLS-K169R/N583R,
   f. MAD7-2xNLS-K169R/Y832K, or
   g. MAD7-2xNLS-K169R/H1025K;
(4) 35S terminator of cauliflower mosaic virus (SEQ ID NO: 82);
(5) ACT3(ACTIN3) (NbACT3) terminator of *Nicotiana benthamiana* (SEQ ID NO: 83); and
(6) Rb7 scaffold/matrix attachment region (MAR) of *Nicotiana tabacum* (SEQ ID NO: 84).

The inserted guide RNA expression cassette was DNA in which the following items were arranged sequentially from the 5' end side:
(7) 35S promoter of cauliflower mosaic virus (SEQ ID NO: 85);
(8) hammerhead (HH) ribozyme (SEQ ID NO: 86);
(9) direct repeat sequence (SEQ ID NO: 87);
(10) a sequence from *Nicotiana benthamiana* XT1 gene as a spacer (SEQ ID NO: 88);
(11) hepatitis delta virus (HDV) ribozyme (SEQ ID NO: 89); and
(12) Nos terminator (SEQ ID NO: 90).

The insertion method is described as follows. A Golden Gate method in which the method of Lampropoulos et al. (2013) was partly modified was used. The cassettes comprising the sequences above were inserted by integrating the cassettes between the BeYDV-derived LIR and SIR sequences by performing a Golden Gate reaction with the NEB Golden Gate Assembly Kit. The reaction conditions were according to the recommended protocol of the kit. As a base sequence (4 bases) for ligation between the cassettes, ACCT was inserted into the immediate upstream of the cassette comprising the sequence 1 above, AACA was inserted between the cassette comprising the sequence 1 above and the cassette comprising the sequence 2 above, GGCT was inserted between the cassette comprising the sequence 2 above and the cassette comprising the sequence 3 above, ACTA was inserted between the cassette comprising the sequences 4 to 6 above and the cassette comprising the sequences 7 to 12 above, and GTAT was inserted into the immediate downstream of the cassette comprising the sequences 7 to 12 above. Further, a base sequence comprising TCAG + 46 bases + CTGC for ligation was inserted between the cassette comprising the sequence 3 above and the cassette comprising the sequences 4 to 6 above.

### Test Example 1: Single-strand Annealing (SSA) Assay

SSA assay was performed to measure genome editing efficiency with the use of each effector expression vector. The overview of this assay is shown in Fig. 1 and described in Production Example 1-1. Specific methods are described below.

### Transfection

Before transfection, HEK293T cells were cultured in a 10-cm dish. HEK293T cells were cultured in Dulbecco's modified Eagle's medium (high-glucose DMEM, FUJIFILM Wako Pure Chemical Corporation) supplemented with 10% fetal bovine serum (FBS, BioSera), 100 units/mL of penicillin, and 100 µg/mL of streptomycin in a 5% CO₂ atmosphere at 37°C.

All plasmids for use in transfection were extracted according to standard protocols using the PureYield Plasmid Miniprep System (Promega K.K.). 100 ng of a reporter vector for measuring genome editing efficiency, 50 ng of Rluc expression vector (pRL-CMV, Promega K.K.) for correcting transfection efficiency, 250 ng of an effector (genome editing tool) expression vector, and 100 ng of a guide RNA expression vector were prepared such that the total amount was 5.5 µL.

Basically, cells on day 2 after passaging were used for transfection. The medium in the Petri dish was discarded. About 2 mL of 1×PBS(-) was added to and spread throughout the Petri dish, and was then aspirated with an aspirator and discarded. Then, 1 mL of trypsin-EDTA (FUJIFILM Wako Pure Chemical Corporation) was added to and spread throughout the Petri dish, and the resulting product was allowed to incubate at 37°C for about 2 minutes. Next, 4 mL of DMEM (containing 10% fetal bovine serum) was added thereto and then collected in a 50-mL centrifuge tube. After centrifugation at 1000 rpm for 3 minutes, the supernatant was removed with an aspirator. An appropriate amount of DMEM (containing 10% fetal bovine serum) was added thereto and suspended, and the cells were counted with a hemocytometer. Subsequently, the cell density was adjusted to 5×10⁵/mL with DMEM (containing 10% fetal bovine serum). 2 µL of HilyMAX (Dojindo Laboratories) was mixed with 10 µL of DMEM, and the mixture was added to 5.5 µL of the plasmid DNA sample, followed by incubation at room temperature for 15 minutes. 100 µL of the cell suspension was added to the plasmid + DMEM + HilyMAX mixture and transferred to a 96-well culture plate (AGC Techno Glass Co., Ltd.). After 24 hours of culture in a 5% CO₂ atmosphere at 37°C, dual-luciferase assay was performed.

### Dual-luciferase Assay

The dual-luciferase assay was performed using the Dual-Glo Luciferase Assay System (Promega K.K.). After 24 hours of transfection, the medium in each well was replaced with 40 µL of 1×PBS(-). 40 µL of the Dual-Glo luciferase reagent was added to each well and mixed sufficiently. After allowing it to incubate at room temperature for 10 minutes, the total amount was transferred to a 96-well luminoplate (PerkinElmer Japan G.K.). Luminescence from firefly luciferase for the Fluc gene expression was measured with a plate reader (PerkinElmer Japan G.K., Nivo multimode plate reader). The Stop & Glo substrate was diluted 100-fold in Dual-Glo Stop & Glo buffer. The diluted solution (40 µL) was added to each well. The resulting mixture was allowed to incubate at room temperature for at least 10 minutes, and luminescence from Renilla luciferase for the Rluc gene expression was measured.

### Data Analysis

Fluc/Rluc values were calculated to correct the difference in transfection efficiency between the assays.

### Test Example 1-1

The test was conducted by using the effector expression vectors obtained in Production Example 1-3 and Production Example 1-4 as the effector expression vectors, and pEXA2J2-hU6-crRNAqa2-NbPDS as the guide RNA expression vector (Test Example 1-1). Fig. 2 shows the configurations of the effector expression vectors used, and Fig. 3 shows the results obtained with the use of each effector expression vector. In both assays, the activity value was highest when pALTER-MAD7-2xNL5 was used.

### Test Example 1-2

The test was conducted by using the effector expression vectors obtained in Production Example 1-5 (pMT-MAD7-2xNLS-[mutation name]) as the effector expression vectors and pEXA2J2-hU6-crRNAqa2GT-NbPDS as the guide RNA expression vector (Test Example 1-2). Fig. 4 shows the results obtained with the use of each effector expression vector. In both assays, the activity value was increased the most when the amino acid mutation of K169R was introduced (2.3-fold increase in both the first assay and the second assay compared with no mutation). In both assays, the second highest increase in the activity value was observed when the amino acid mutation of H1025R was introduced (2-fold increase in the first assay and 1.9-fold increase in the second assay compared with no mutation). Other mutations with which the activity value increased 1.5-fold or more in both assays were N583K, N583R, Y832R, H1025K, and C1219N.

### Test Example 1-3

The test was conducted by using the effector expression vectors obtained in Production Example 1-6 (pMT-MAD7-2xNLS-[mutation name]) as the effector expression vectors and pEXA2J2-hU6-crRNAqa2GT-NbPDS as the guide RNA expression vector (Test Example 1-3). Fig. 5 shows the results obtained with the use of each effector expression vector. An increase of about 2-fold in the activity value was observed compared with no mutation when the amino acid mutation of K169R was introduced. When a combination of K169R with any one of amino acid mutations of S469R, N583K, N583R, Y832K, Y832R, H1025K, and H1025R was introduced, additive effects (an increase of about 2.2-fold to 2.4-fold compared with no mutation) were observed.

### Test Example 1-4

The test was conducted by using the effector expression vectors obtained in Production Example 1-7 and Production Example 1-8 as the effector expression vectors and pEXA2J2-hU6-crRNAqa2GT-NbPDS as the guide RNA expression vector (Test Example 1-4). Fig. 6 shows the configurations of the effector expression vectors used, and Fig. 7 shows the results obtained with the use of each effector expression vector. An increase in the activity value was observed when the T5 exonuclease with 3xFLAG+SV40 NLS was fused to the N-terminus of the MAD7 (MAD7-2xNLS), in which two NLSs (bipartite + monopartite) were added to the C-terminus. A further increase in the activity value was observed when a single mutation and multiple mutations were introduced into the MAD7 protein portion above.

### Test Example 2: Agroinfiltration Assay Using Tobacco

A genome editing test was conducted in a plant by using the effector expression vector of Production Example 2, and the resulting genome editing efficiencies were compared. The details are described below.

### Preparation of Agrobacterium

The effector expression vector of Production Example 2 was introduced into *Rhizobium radiobacter* strain GV3101 by electroporation, and the resulting product was spread on LB plates containing antibiotics, followed by culturing at 28°C for 2 to 3 days. Single colonies were pre-cultured overnight (28°C, 180 rpm) in 3 mL of LB liquid medium containing antibiotics. After addition of 7 mL of LB liquid medium and culturing for 2 hours (28°C, 180 rpm), the bacteria were collected. The collected bacteria were resuspended in an induction buffer (10 mM MES, 100 µM acetosyringone, pH of 5.5 with NaOH), and induction was performed for 2 to 3 hours. After the resulting product was resuspended in an infiltration buffer (5 mM MES, pH of 5.5 with NaOH), each Agrobacterium liquid was adjusted such that OD=0.7 to obtain bacterial liquids for inoculation.

### Infiltration into Tobacco Leaves

After seeds of *Nicotiana benthamiana* for use were sterilized with Haiter, the seeds were spread on a seeding medium, allowed to incubate in the dark at 4°C for 4 days, and then allowed to grow for 1 week in a chamber at 25°C with a cycle of 16 hours of light and 8 hours of dark (16L8D). The seedlings were planted in culture soil obtained by mixing Hana-chan Culture Soil (Hanagokoro) and vermiculite at 1:1 and allowed to grow under the conditions of 26°C to 30°C and 16L8D. Individuals obtained 2 to 3 weeks after transplanting were used for infiltration. Infiltration was performed from the underside of the leaf by using a 1-mL syringe. After inoculation, the plant was kept moist to prevent drying and was allowed to grow in an environment at 26 to 30°C and 16L8D.

### Mutation Detection by CAPS (Cleaved Amplified Polymorphic Sequence)

Seven days after inoculation, the inoculation sites were cut out with an 8-mm-diameter punch and grinded with a MB2200 Multi-beads shocker (Yasui Kikai Corporation), and the total DNA was extracted using a Maxwell automated nucleic acid extraction system (Promega Corporation). Using the extracted DNA as a template, the DNA region containing the target sequence was PCR-amplified with KOD Fx Neo (TOYOBO Co., Ltd.). The PCR products were column-purified (Macherey-Nagel) and then treated with EcoRI (NEB), and the presence or absence of mutation introduction was detected with the MultiNA microchip electrophoresis system for DNA/RNA analysis (SHIMADZU Corporation). The presence of an uncleaved band indicates the occurrence of genome editing. The percentage (%) of the uncleaved bands was calculated with respect to the percentage (uncleaved band + cleaved band) taken as 100%, and was defined as genome editing efficiency.

Fig. 8 shows the results obtained with the use of each effector expression vector. When the amino acid mutation of K169R was introduced, an increase of about 2.3-fold in editing efficiency was observed compared with the case without mutation. Similarly, when the amino acid mutation of N583R was introduced, an increase of about 2-fold in editing efficiency was observed compared with the case without mutation. When a combination of amino acid mutations of K169R with S469R or H1025K was introduced, a further increase was observed in editing efficiency (an increase of about 3.7-fold to 4-fold compared with the case without mutation). No uncleaved band was observed in those of wild type in which an effector expression vector was not used.

## Claims

1. A protein comprising
an amino acid sequence b1 of MAD7 protein,
wherein the amino acid sequence b1 is a mutant sequence of the amino acid sequence represented by SEQ ID NO: 6 and comprises a K169R mutation in the amino acid sequence represented by SEQ ID NO: 6.

2. The protein according to claim 1, wherein the amino acid sequence b1 further comprises at least one mutation selected from the group consisting of S469R and H1025K in the amino acid sequence represented by SEQ ID NO: 6.

3. The protein according to claim 1 or 2, comprising
(b) a MAD7 domain comprising the amino acid sequence b1 of MAD7 protein and a concatenated sequence b2 of multiple nuclear localization signal sequences.

4. The protein according to claim 3, wherein in the MAD7 domain, the concatenated sequence b2 is located on the C-terminal side of the amino acid sequence b1.

5. The protein according to claim 3 or 4, comprising:
(a) a 5' to 3' exonuclease domain comprising an amino acid sequence a1 of 5' to 3' exonuclease and a nuclear localization signal sequence a2; and
(b) a MAD7 domain comprising the amino acid sequence b1 of MAD7 protein and a concatenated sequence b2 of multiple nuclear localization signal sequences.

6. The protein according to claim 5, wherein the 5' to 3' exonuclease domain is located on the N-terminal side of the MAD7 domain.

7. The protein according to claim 5 or 6, wherein in the 5' to 3' exonuclease domain, the nuclear localization signal sequence a2 is located on the N-terminal side of the amino acid sequence a1.

8. The protein according to any one of claims 5 to 7, wherein the nuclear localization signal sequence a2, the amino acid sequence a1, the amino acid sequence b1, and the concatenated sequence b2 are arranged in this order from the N-terminal side.

9. The protein according to any one of claims 1 to 8, wherein the amino acid sequence b1 is a mutant sequence of the amino acid sequence represented by SEQ ID NO: 6 and comprises at least one mutation selected from the group consisting of A24E, I180K, A290R, S469K, S469R, K535R, N583K, N583R, K590R, I646K, I646R, T714K, T714R, N827D, Y832K, Y832R, K970R, H1025K, H1025R, S1175A, and C1219N in the amino acid sequence represented by SEQ ID NO: 6.

10. The protein according to claim 9, wherein the mutation is at least one mutation selected from the group consisting of N583K, N583R, Y832K, Y832R, H1025K, H1025R, and C1219N.

11. A protein comprising:
(a) a 5' to 3' exonuclease domain comprising an amino acid sequence a1 of 5' to 3' exonuclease and a nuclear localization signal sequence a2; and
(b) a MAD7 domain comprising an amino acid sequence b1 of MAD7 protein and a concatenated sequence b2 of multiple nuclear localization signal sequences.

12. A protein comprising
an amino acid sequence b1,
wherein the amino acid sequence b1 is a mutant sequence of the amino acid sequence represented by SEQ ID NO: 6 and comprises at least one mutation selected from the group consisting of A24E, K169R, I180K, A290R, S469K, S469R, K535R, N583K, N583R, K590R, I646K, I646R, T714K, T714R, N827D, Y832K, Y832R, K970R, H1025K, H1025R, S1175A, and C1219N in the amino acid sequence represented by SEQ ID NO: 6.

13. A protein comprising:
(ai) a 5' to 3' exonuclease domain comprising an amino acid sequence a1 of 5' to 3' exonuclease and a nuclear localization signal sequence a2; and
(bi) a MAD7 domain comprising an amino acid sequence b1 of MAD7 protein.

14. A polynucleotide comprising a coding sequence for the protein of any one of claims 1 to 13.

15. A polynucleotide comprising:
(aii) an expression cassette of a protein comprising a 5' to 3' exonuclease domain comprising an amino acid sequence a1 of 5' to 3' exonuclease and a nuclear localization signal sequence a2; and
(bii) an expression cassette of a protein comprising a MAD7 domain comprising an amino acid sequence b1 of MAD7 protein and a concatenated sequence b2 of multiple nuclear localization signal sequences.

16. A cell comprising the polynucleotide of claim 14 or 15.

17. A composition for genome editing, comprising at least one member selected from the group consisting of the protein of any one of claims 1 to 13 and the polynucleotide of claim 14 or 15.

18. A genome editing method, comprising introducing at least one member selected from the group consisting of the protein of any one of claims 1 to 13 and the polynucleotide of claim 14 or 15 into a cell or a non-human organism.

19. A method for producing a genome-edited cell or genome-edited non-human organism, comprising introducing at least one member selected from the group consisting of the protein of any one of claims 1 to 13 and the polynucleotide of claim 14 or 15 into a cell or a non-human organism.
